(19) Europäisches Patentamt

European Patent Office

Office européen des brevets

(11) **EP 0 969 744 B1**

(12) **EUROPEAN PATENT SPECIFICATION**

(45) Date of publication and mention
of the grant of the patent:
**21.05.2003 Bulletin 2003/21**

(51) Int Cl.7: **A23L 1/302**

(86) International application number:
**PCT/CA98/00286**

(21) Application number: **98912176.9**

(22) Date of filing: **25.03.1998**

(87) International publication number:
**WO 98/043617 (08.10.1998 Gazette 1998/40)**

(54) **NUTRITIONAL COMPOSITION FOR IMPROVEMENTS IN CELL ENERGETICS**

ERNÄHRUNGSZUSAMMENSETZUNG ZUR VERBESSERUNG DER ZELLENENERGIE

COMPOSITION NUTRITIONNELLE SERVANT A AMELIORER LA CAPACITE ENERGETIQUE
CELLULAIRE

(84) Designated Contracting States:
**AT BE CH DE DK ES FI FR GB GR IE IT LI LU MC
NL PT SE**

(30) Priority: **27.03.1997 US 826324
06.01.1998 US 2765**

(43) Date of publication of application:
**12.01.2000 Bulletin 2000/02**

(73) Proprietors:
• **Sole, Michael J.**
**Toronto, Ontario M6B 1L3 (CA)**
• **Jeejeebhoy, Khursheed N.**
**Toronto, Ontario M4V 2V5 (CA)**

(72) Inventors:
• **Sole, Michael J.**
**Toronto, Ontario M6B 1L3 (CA)**
• **Jeejeebhoy, Khursheed N.**
**Toronto, Ontario M4V 2V5 (CA)**

(74) Representative: **van Westenbrugge, André et al
Nederlandsch Octrooibureau
P.O. Box 29720
2502 LS The Hague (NL)**

(56) References cited:
**EP-A- 0 129 418          EP-A- 0 659 349
WO-A-89/01740          WO-A-94/14458
GB-A- 2 230 418          US-A- 4 599 232**

• **DATABASE WPI Section Ch, Week 9608 Derwent
Publications Ltd., London, GB; Class B05, AN
96-074741 XP002079121 -& JP 07 330584 A
(TAISHO PHARM CO LTD) , 19 December 1995**
• **DATABASE WPI Section Ch, Week 9122 Derwent
Publications Ltd., London, GB; Class B04, AN
91-159781 XP002079122 -& JP 03 094655 A
(OTSUKA PHARM CO LTD) , 19 April 1991**

Remarks:
The file contains technical information submitted
after the application was filed and not included in this
specification

Note: Within nine months from the publication of the mention of the grant of the European patent, any person may give
notice to the European Patent Office of opposition to the European patent granted. Notice of opposition shall be filed in
a written reasoned statement. It shall not be deemed to have been filed until the opposition fee has been paid. (Art.
99(1) European Patent Convention).

**Description**

## TECHNICAL FIELD OF THE INVENTION

There are four critical organ systems that are especially likely to fail in aging and critical illness, namely the cardiovascular system, the central nervous system, the musculoskeletal and the immune systems. Defective mitochondrial energy production, and the resulting increased levels of free radicals are believed to play a central role.

Failure of the cardiovascular system, particularly congestive heart failure, has emerged as a major health problem during the past two decades and now affects approximately 1% of the population of the United States and Canada. This increase reflects both the aging of the population and the success of modern cardiovascular medicine in converting acute, often previously fatal, cardiac diseases into a more chronic process.

The underlying abnormality in congestive heart disease is myocardial dysfunction, which leads to inadequate blood flow to peripheral tissues. Although there have been considerable advances in our understanding of the pathogenesis of heart failure in recent years, critical questions remain about the evolution of cardiac dysfunction to terminal failure. In spite of recent advances in treating cardiac disease, no presently available therapeutic intervention has been shown to substantially improve the long-term survival of patients with dilated cardiomyopathy and congestive heart failure; myocardial failure is relentlessly progressive in almost all patients who develop overt symptoms.

This failure to develop an effective therapy may be attributable to the fact that several metabolic abnormalities have been found in the failing myocardium, which together, as indicated below, result in progressive loss of cardiac myocytes (muscle)

There is a progressive accumulation of calcium in the muscle, resulting in turn in increased calcium in the mitochondria. This increase, together with the basic cardiac disease (ischemic, viral, toxic, genetic) decrease myocyte energy production and increase oxidative stress, which in turn results in free radical damage. The combined result of these three processes promotes myocyte dysfunction and death. In addition, these processes also influence skeletal muscle and contribute to fatigue and disability in the patient, reducing the overall health of the patient.

The modern pharmacological therapy of heart failure has focused on the amelioration of fluid overload and haemodynamic abnormalities, but has not addressed the fundamental problem of progressive loss of cardiac muscle. The inexorable myocyte loss by apoptosis is believed to be the key factor responsible for myocardial decompensation, and the ultimate demise of the patient. Oxidative stress, calcium overload and cellular energy deficiency have all been shown to be principal stimuli for the development of apoptosis.

Nutritional deficiencies have increasingly been recognized as being among the factors that aggravate cardiac and skeletal muscle dysfunction. For example, there is a relationship between protein deficiency and mitochondrial dysfunction in muscle. Several nutritional agents, including creatine, carnitine and taurine have been shown to improve muscle performance.

The presence of protein-energy malnutrition among heart failure patients has been found to be greater than that of patients with cancer, alcoholism or acute infection. This malnutrition is due to both reduced intake and increased energy demands.

Heart failure itself contributes to these deficiencies, while at the same time imposing increased nutritional requirements on the patient. Cardiac failure often results in a cascade of metabolic effects such as tissue hypoxia, anorexia, hypermetabolism, weakness, dyspnea and hypomotility of the gastrointestinal tract, all leading to poor nutrient intake, which in turn can be aggravated by drugs commonly prescribed for heart disease. These factors lead to compromised food and nutrient intake, and subsequently contribute to the poor nutritional status of cardiac patients.

These deficiencies impair myocardial energy metabolism and protein synthesis, and increase intracellular calcium and oxidative stress in the failing myocardium. Skeletal muscle is similarly impaired.

Thus, nutritional deficiency is an integral contributor to myocyte dysfunction and loss as welt as the fatigue and exercise intolerance seen in heart failure.

Adequate myocyte nutrition, through dietary supplementation, is essential to any therapeutic strategy designed to benefit patients with heart failure.

The recommended daily allowances (RDA) for vitamins and related micronutrients established by federal nutrition authorities in Canada, the United States and Western Europe (e.g., The Canada Food Guide) are often relied upon by doctors and other health care professionals to determine the nutritional requirements of their patients. However, the recommended nutritional requirements are commonly determined through the analysis of deficiency data from otherwise healthy humans and animals. We have found that they cannot be relied upon for determining the nutritional requirements of patients suffering from cardiac or other diseases, or predisposed to sickness through genetic constitution.

## BACKGROUND ART

The role of some nutrients in maintaining good health generally and in treating various disorders, has long been recognized, particularly in the field of so-called "alternative" medicine, which often emphasizes the use of "natural" ingredients for these purposes. Many nutrients have been studied, and much research has been directed to the possible benefits to be derived from supplementing diet with individual nutrients. Nothing in the art, however, has suggested that there is a synergistic benefit to be gained through the use of a combination of nutrients. For example:

JP-A-7330584 (Taisho Pharm Co Ltd) discloses a composition containing ubiquinone, carnitine and taurine for prevention and recovery from fatigue.

JP-A-3094655 (Otsuka Pharm Co Ltd) is a nutritional supplement composition for treating degenerate diseases. The supplement includes hydrocarbons, proteins, fats and dietary fibres.

WO 89/01740 (Sockerbolaget) is a composition intended as a diet fortification to increase the efficiency of muscle work which includes ubiquinone as the essential component.

GB-A-2230418 (Therapeutic health Advisory Services Limited) is a health supplement for humans including coenzyme Q10 and carnitine.

US patent 4,599,232 (Bertelli) is a pharmaceutical composition containing carnitine and coenzyme Q10 in rations from 100:1 to 2:1. It is for treatment of disorders such as atherosclerosis, myocardial insufficiency and hypertension.

EP 0129418 (Glaxo Group) discloses an infant food for use in the feeding of low birth weight infants which include vitamins, taurine and carnitine.

WO 94/14458 (Abbott Laboratories) relates to a generic powder base high in fats, carbohydrates, vitamins, minerals and trace elements which can be readily mixed with amino acids to provide therapeutic products intended for use in nutritional support of infants.

EP 0659349 relates to methods for lowering glucose levels of diabetics by administering myo-inositol. Some compositions include carnitine and taurine.

These compositions are not satisfactory for treatment of a broad range of diseases. There is a need for a nutritional supplement which addresses the underlying metabolic disorders to treat and prevent a broad range of diseases, disorders and abnormal physical states.

Nutrient deficiency in organs is not readily detected, as nutrient levels in the blood (which can be relatively easily determined) are often significantly higher than levels of the same nutrient in critical organ systems. Consequently, analysis of blood levels of particular nutrients may be misleading and is consequently not commonly done. Thus, nutrient deficiency as a significant cause of the failure of critical organ systems has not been well-recognized in either traditional or alternative medicines. Doctors rarely prescribe or recommend supplements of even one nutrient to deal with the failure of critical organ systems.

We have found that inadequate nutritional substrate is a cause of impaired cell energetics and that nutrition can be used to prevent or delay the onset of cardiac failure, and failure of the other critical organ systems identified above, promoting recovery of patients from disease states affecting the heart and critical organ systems. The treatment of heart diseases, especially congestive heart failure, by adjunctive treatment with a nutritional component is described in the following articles.

Hofman-Bang C et al. ('Coenzyme Q10 as an adjunctive in the treatment of chronic congestive heart failure', J Card Fail Vol. 1 No. 2 1995, pages 101-107) have reported the results of a study into the effect of coenzyme Q10 on patients with stable chronic congestive heart failure. The results obtained indicate that oral long-term treatment with 100 mg coenzyme Q10 only slightly improves maximal exercise capacity and the quality of life.

Azuma J et al. ('Therapeutic effect of taurine in congestive heart failure: a double blind crossover trial', Clin Cardiol, Vol. 8, pages 276-282, May 1985) have investigated the therapeutic effect of taurine in congestive heart failure. The results indicate that addition of taurine to conventional therapy is safe and effective for the treatment of patients with congestive hear failure.

Mancini et al. ('Controlled study on the therapeutic efficacy of prapionyl-L-carnitine in patients with congestive heart failure', Arneim.-Forsch./Drug Res 42(II), Nr. 9, pages 1101-1104) have reported the results of a study on the ther apeutic effect of propionyl-L-carnitine in patients with congestive heart failure. It is concluded that propionyl-L-carnitine represents a drug of undoubted therapeutic interest in patients with congestive heart failure, in whom it could be efficaciously administered along with a standard pharmacological thereapy.

We have determined that adequate myocyte nutrition is essential to any therapeutic strategy designed to benefit patients with heart failure. There is a need for a nutritional supplement that can be taken by persons suffering from illness or with a genetic predisposition to illness.

This has led us to invent a composition for the improvement of mitochondrial energetics. Other investigators have used certain of the nutrients in our supplement alone to treat heart failure and other diseases. However, we have found that the replacement of only one of the key nutritional constituents will not correct the connected abnormalities in multiple parts of the myocardial bioenergetic pathway which is deranged in cardiac failure, but merely treats a problem at a single point in a pathway. There has been no appreciation of the core of nutrients essential for good mitochondrial function, and of the synergistic effect obtained from these core ingredients in treating cardiac failure and diseases of other critical organ systems. The use of single nutrients has given mixed, often conflicting, results.

## DISCLOSURE OF THE INVENTION

We have determined that what is required to correct the cascading series of metabolic abnormalities in patients with myocardial dysfunction and other diseases mentioned above, is a nutritional supplement that can replenish certain key nutrients and influence several metabolic pathways which interact to subserve mitochondrial function..

Replenishment of protein is important as well. Protein-calorie malnutrition has been found to contribute to both skeletal and cardiac muscle dysfunction in patients with cardiac failure. It has been shown that in malnutrition there is a change in muscle membrane potential, resulting in reduced intracellular ionic potassium. The reduced cellular potassium cannot be simply corrected by giving potassium but requires restitution of nutrition. Studies have suggested that malnutrition is associated with a reduced rate of oxidative phosphorylation, suggesting a mitochondrial abnormality.

Skeletal muscle function, including that of the diaphragm, can be rapidly altered by nutrient deprivation and can be restored by refeeding. Changes in muscle function have been shown to be specific to alterations in the nutritional status and not influenced by sepsis, trauma, renal failure and steroid administration.

We have determined that it is important to correct protein-calorie malnutrition, with an emphasis on protein repletion, in order to obtain the maximum functional benefits of administering cardiac specific micronutrients, including the nutrient formulation of the present invention. Current diet supplementing strategies for correcting protein-calorie malnutrition focus on giving supplements of protein and energy (carbohydrates and fats). No supplement to date has addressed the cascading series of metabolic abnormalities that can lead to mitochondrial dysfunction. We have shown that in the skeletal muscle, protein-calorie, malnutrition profoundly reduces mitochondrial oxidative phosphorylation, and reduces calcium cycling in cardiac muscle. Protein feeding has been found to restore rapidly any abnormality due simply to protein-energy malnutrition.

The invention includes a nutritional supplement in a form suitable for at least once daily administration, comprising at least 0.6 g of L-Carnitine or its functional analogue selected from the group consisting of Acetyl-1-carnitine, Propionyl-1-carnitine and mixtures thereof, at least 30 mg of Coenzyme Q10 (Ubiquinone) or its functional analogues, and Taurine or its precursors. The supplement may further comprise one or more nutrients selected from the group consisting of Cysteine, Creatine, Vitamin E (RRR-d-alpha-tocopherol), Vitamin C (ascorbic acid), Selenium, and Thiamine. The nutritional supplement may further comprise Cysteine, Creatine, Vitamin E (RRR-d-alpha-tocopherol), Vitamin C (ascorbic acid), Selenium, and Thiamine. The supplement may inlcude a protein-enriched formulation. It may be adapted to be administered in an amount effective for the treatment of heart disease, disorder or an abnormal physical state.

In a variation, the nutritional supplement includes an form suitable for at least once daily administration including from about 0.6 to about 6.0 gm of L-Carnitine or its functional analogues, from about 30 to about 300 mg of Coenzyme Q10 (Ubiquinone), and from about 0.5 to about 5g of Taurine or its precursors. The supplement may be in a form suitable for at least once daily administration and include at least about 0.75 g of L-Carnitine or its functional analogues, at least about 25 mg of Coenzyme Q10 (Ubiquinone), at least about 0.75 g of Taurine or its precursors, and when present in the formulation at least about 0.1 g of Cysteine, at least about 1 g of Creatine, at least about 100 IU of Vitamin E (RRR-d-alpha-tocopherol), at least about 100 mg Vitamin C (ascorbic acid), at least about 8 mcg. of Selenium and at least about 10 mg. Thiamine. The nutritional supplement may be in a form suitable for at least once daily administration including from about 0.6g to 6.0 gm of L-Carnitine or its functional analogue, from about 30 mg to about 300 mg of Coenzyme Q10 (Ubiquinone), from about 0.5g to about 5g of Taurine or its precursors, and when present in the supplement, from about 0.1g to about 1 g of Cysteine, from about 0.5 to about 5g of Creatine, from about 100 to about 1200 IU of Vitamin E (RRR-d-alpha-tocopherol), from about 100 to about 1000 mg Vitamin C (ascorbic acid), from about 8mcg to about 50mcg Selenium, from about 10mg to about 100 mg Thiamine.

The nutritional supplement may be in a form suitable for once daily administration, including at least about 0.75 g L-Carnitine or its functional analogues, and at least about 0.75 g. Taurine or its precursors. The nutritional supplement may be in a form suitable for at least once daily administration, including at least 5 g. L-Carnitine or its functional analogues, 200 mg Coenzyme Q10 (Ubiquinone) or its functional analogues, 3 g. Taurine and 3 g. Creatine. The nutritional supplement is also useful in a form suitable for multiple doses per day. The nutritional supplement can also be adapted to be administered in amounts effective for the treatment of cardiovascular disease, disorder or condition. The nutritional supplement may be in an amount effective for the treatment of a disease, disorder or abnormal physical state selected from the group consisting of heart disease (including congestive heart failure), neurodegenerative disease, immune diseases, stroke, AIDS, chronic multisystem disease, respiratory muscle fatigue such as chronic obstructive lung disease, lung or renal disease, chronic fatigue syndrome, effects of immunosuppressive drugs or chemotherapy, wasting, cachexia from cancer or sepsis. The nutritional supplement may be in an amount effective for administration to mammals for the purpose of enhancing neuromuscular or athletic performance. The nutritional supplement of claims may be adapted to be administered to mammals, including humans, in amounts effective for correcting diseases, conditions and infirmities due to aging. The nutritional supplement can be delivered in a protein enriched formulation including a liquid dairy based product, a dehydrated dairy based product, a soy based product, and a bar. The nutritional supplement may be in the a form of a nutritional protein and calorie enriched bar.

The invention also includes the use of the nutritional supplement of in the treatment of critical organ systems in mammals. The nutritional supplement may further includes one or more nutrients selected from the group consisting of Cysteine, Creatine, Vitamin E (RRR-d-alpha-tocopherol), Vitamin C (ascorbic acid), Selenium, and Thiamine. The critical organ system is preferably the cardiovascular system. The use may be in the treatment of congestive heart failure. The invention includes a method of treating a critical organ system in mammals by administering a nutritional supplement including L-Carnitine or its functional analogues Coenzyme Q10 (Ubiquinone), and Taurine or its precursors.

In the method, said nutritional supplement may further comprise one or more nutrients selected from the group consisting of Cysteine, Creatine, Vitamin E (RRR-d-alpha-tocopherol), Vitamin C (ascorbic acid), Selenium, and Thiamine. The invention includes a method of treating congestive heart failure by administering on a regular basis a nutritional supplement including from about 0.6 to about 6.0 gm of L-Carnitine or its functional analogues, from about 30 to about 300 mg of Coenzyme Q10 (Ubiquinone), and from about 0.5 to about 5g of Taurine or its precursors. The nutritional supplement may further include from about 0.1g to about 1.0g of Cysteine, from about 0.5g to about 5g of Creatine, from about 100 to about 1200 IU of Vitamin E (RRR-d-alpha-tocopherol), from about 100mg to about 1000mg of Vitamin C (ascorbic acid) from about 8 mcg, to about 50 mcg. of Selenium, and from about 10mg to about 100 mg Thiamine.

The invention also includes the use, in a single or divided daily dose, of an effective amount of carrier and L-Carnitine or its functional analogue, Coenzyme Q10 (Ubiquinone) and Taurine or its precursors for preparation of a nutritional supplement for treatment of heart disease (including congestive heart failure), functional deterioration associated with ageing, neurodegenerative disease, immune disease, stroke, AIDS, chronic multisystem disease, respiratory muscle fatigue, chronic obstructive lung disease, lung disease, renal disease, chronic fatigue syndrome, effects of immunosuppressive drugs on patients, effects of chemotherapy drugs on cancer patients, wasting, cachexia and a disease, disorder or abnormal physical state including impaired cellular nutrition and mitochondrial energetics and increased oxidative stress. The use as claimed comprises an effective amount of a carrier and L-Carnitine, Coenzyme Q10 (Ubiquinone) and Taurine. The amounts that may be used, as well as the carriers and additional compounds that may be added for the uses of the invention are as described above with respect to the nutritional supplement.

## BRIEF DESCRIPTION OF THE DRAWINGS.

Figure I is a schematic diagram of the interaction of the nutrients included in this invention and their interaction in cell energetics.
Figure 2 shows electron micrographs of heart specimens from hamsters in Experiment 1.
Figure 2(a) shows a normal hamster heart. Figure 2(b) shows a control or non-treated cardiomyopathic heart; and Figure 2(c) shows a heart of a hamster treated with a nutritional supplement of the present invention.
Figure 3 shows the developed pressure and rate of pressure development (dp/dt) of hamster hearts (Langendorff Model) in Experiment 1. Figure 3(a) shows a normal hamster heart;

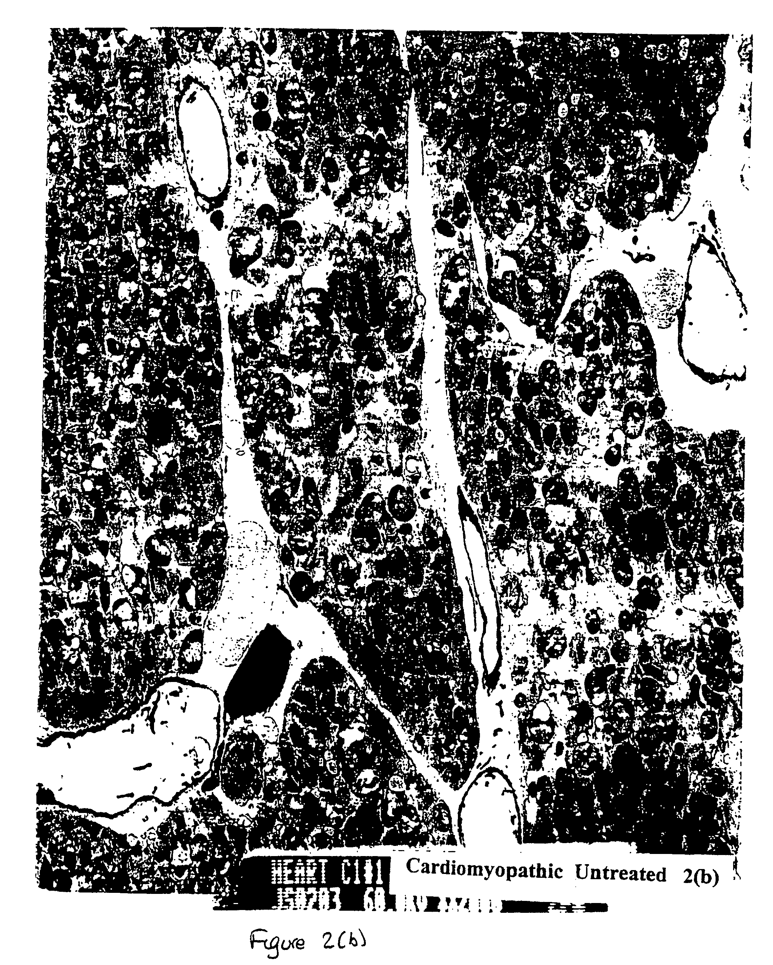

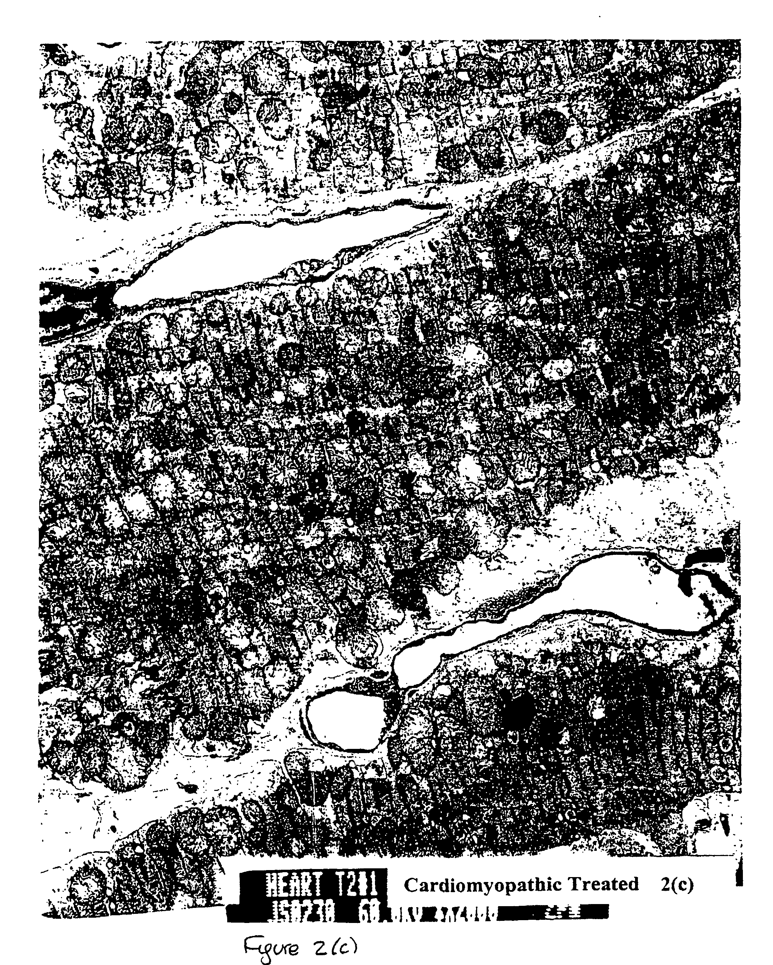

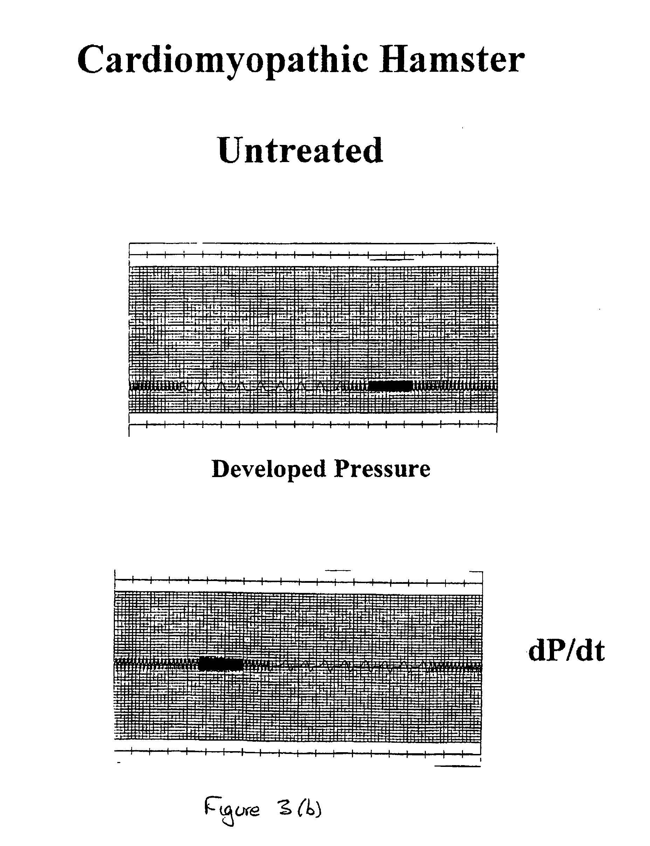

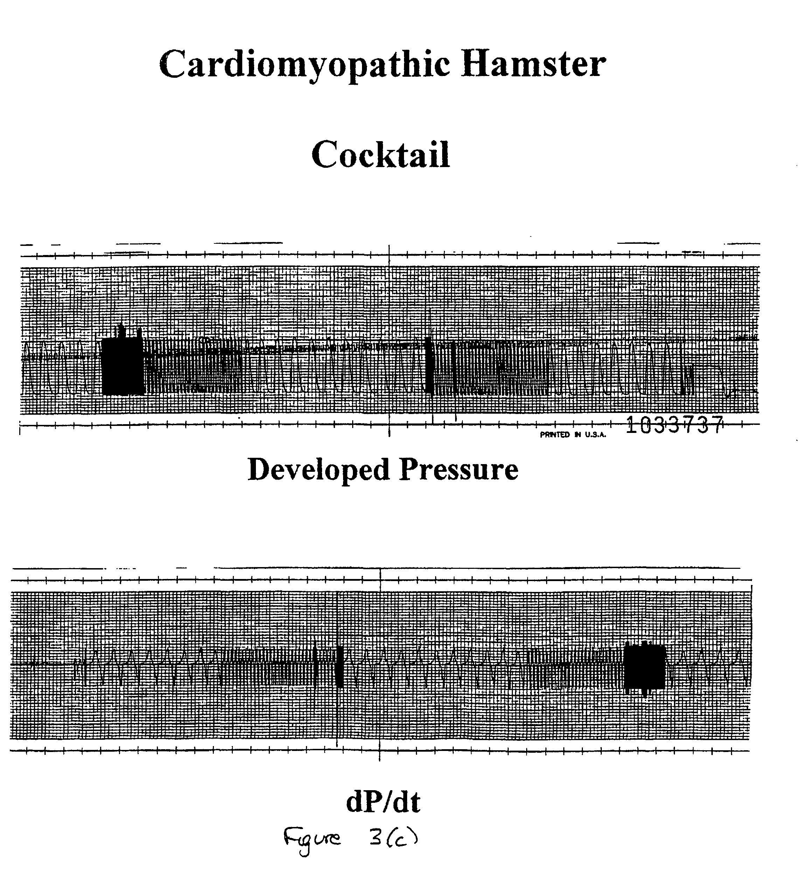

Figure 2(b) shows a control non-treated cardiomyopathic heart; Figure 3(c) shows a heart of a hamster treated with a nutritional supplement of the present invention.

## MODES FOR CARRYING OUT THE INVENTION

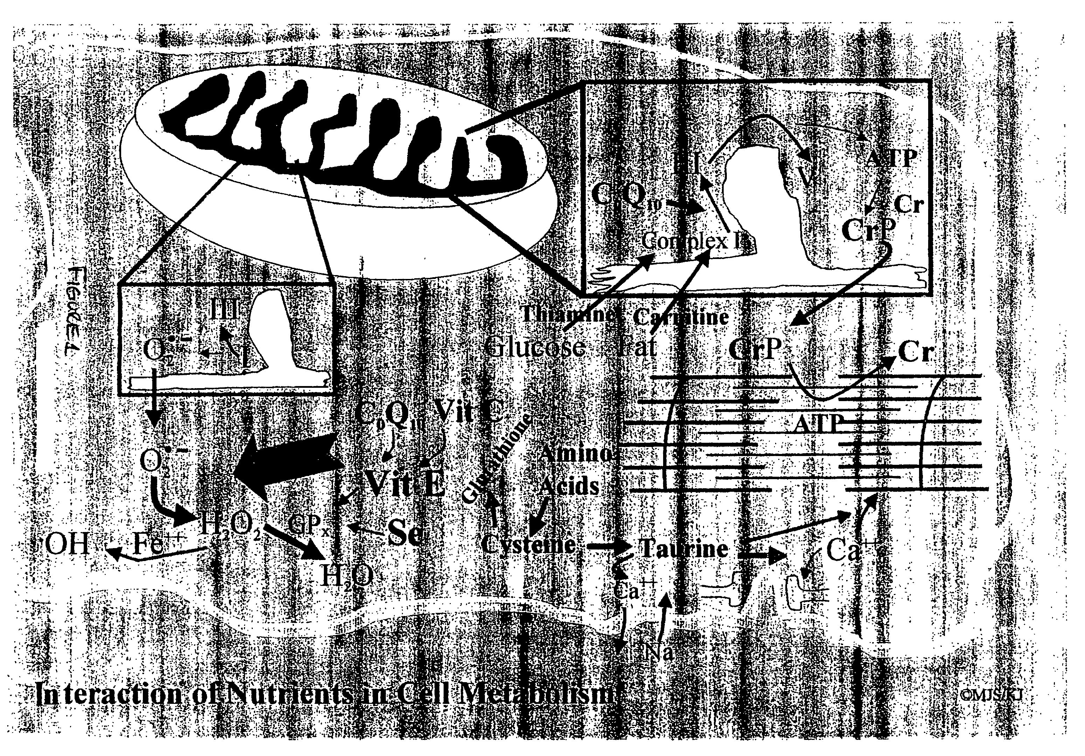

Researchers have up to now not recognized that optimizing mitochondrial function depends upon the synergistic correction of cellular and mitochondrial energy substrates as demonstrated graphically in Figure 1, which will lead to improved energetics, reduced oxidative stress and better calcium homeostasis, thus providing a synergistic response clinically beneficial to the patient.

The present invention provides a nutritional supplement comprising three core ingredients which when administered together provide a synergistic effect over the use of such nutrients individually, and acts to a nutritional supplement that maintain or restore mitochondrial function and to prevent cardiac failure or aid recovery from cardiac disease. In addition it can also aid in the management of neurodegenerative, musculoskeletal diseases including the muscular abnormality in chronic obstructive lung disease (COPD) and immune disorders. The combination of nutrients of this invention addresses what we have found to be an interrelated series of disruptions in cellular metabolism, that are present in heart failure and conditions such as aging, chronic neurodegenerative disease, immune diseases such as AIDS, chronic multisystem disease, chronic lung or renal disease, chronic fatigue syndrome, patients on immunosuppressive drugs post-transplantation, cancer patients on doxorubicin or related drugs, wasting or cachexia from cancer or sepsis, and in normal humans wishing better neuromuscular or athletic performance, and thus provides more reliable, effective treatment of these conditions.

The nutritional supplement is preferably delivered orally in liquid form, but other methods of administration such as intravenous administration may be used.

The invention is a nutritional supplement that helps to correct or prevent the cascading series of metabolic abnormalities responsible principally for cardiac disease, but will have a similar effect on neuromuscular, central nervous and immune system dysfunction in a wide variety of diseases. Rather than merely addressing problems at particular points in metabolic pathways, the nutritional supplement of the invention uses a holistic approach to restoring and improving function at many points in cell metabolism, for example at multiple points along the mitochondrial bioenergetic pathway. The effectiveness of this nutritional supplement in preventing and correcting myocardial dysfunction has been demonstrated in vivo (Example 1).

This invention relates to a dietary supplement comprising effective amounts of L-Carnitine (or its functional analogues Acetyl-Carnitine or Proprionyl-1-Carnitine), Coenzyme Q10 (Ubiquinone) and Taurine as its core constituents. These three nutrients have been found by us to be essential for the correction of the abnormality in mitochondrial energetics in cardiac failure and the different diseases referred to above. In one preferred embodiment, the dietary supplement will comprise, in addition to the core ingredients, one or more of Cysteine, Creatine, Vitamin E (RRR-d-alpha-tocopheryl), Vitamin C (ascorbic acid), Selenium, and Thiamin, and may also contain other more conventional nutrients to provide a high protein nutritional feeding.

In another preferred embodiment, this invention relates to a dietary supplement taken in a high protein formulation such as a dairy based drink, a dehydrated dairy product, a soya based drink or a dehydrated product, or a nutritional bar containing: L-Carnitine or its functional analogues Acetyl- and Proprionyl-1-Carnitine, Coenzyme Q10 (Ubiquinone), Taurine. The addition of one or more of Cysteine, Creatine, Vitamin E (RRR-d-alpha-tocopheryl), Vitamin C (ascorbic acid), Selenium, and Thiamine will aid the action of the core constituents.

One particularly preferred embodiment of the present invention provides a dietary supplement in the form of a high protein, high calorie formulation in the form of a drink and containing: from 0.5-5g, preferably about 3g, of L-Carnitine or its functional analogues Acetyl- and Proprionyl-1-Carnitine; 30-200mg of Coenzyme Q10 (Ubiquinone), 0.1-3g. of Taurine; 0.5gm-1.5g of Cysteine; 2.5g of Creatine, about 600 IU of Vitamin E (RRR-d-alpha-tocopheryl), about 1000mg of Vitamin C (ascorbic acid), about 50mcg of Selenium, and 25mg Thiamine. These doses may vary from 25% to 300% of the foregoing amounts for specialized formulations, and represent typical average daily dosages for humans with critical organ failure.

In another particularly preferred embodiment, the same nutrients are incorporated into a nutritional bar together with protein supplements, and other ingredients such as sugars and grains commonly used to make nutritional bars. The amount of nutrients in each bar may be varied depending on the number of bars intended to be taken by the patient each day.

This formulation ensures a high quality protein to optimize muscle function so as to allow the above nutrients in combination to synergistically interact for the benefit of the patient - that is the effect of all of the ingredients combined will be greater than the sum of the individual parts as they address a cascading series of metabolic abnormalities. There is a core of specific nutrients, which must be combined to be effective, and a larger number for optimal effectiveness. Conversely omission of the core will detract from the overall efficacy of this supplement. In addition to maintaining protein stores, the supplement corrects abnormalities in: (a) myocardial energetics, (b) intracellular calcium and (c) oxidative stress.

This supplement also benefits patients, with or without heart failure, with other conditions in which cellular nutrition, mitochondrial energetics and function are impaired or less than desired and oxidative stress is increased, including but not exclusively for musculoskeletal, immune and disorders of the central nervous system especially those related to aging. Such disorders include neurodegenerative disease, immune diseases, stroke, AIDS, chronic multisystem disease, respiratory muscle fatigue such as chronic obstructive lung disease, lung or renal disease, chronic fatigue syndrome, patients on immunosuppressive drugs, cancer patients treated with drugs such as doxorubicin, wasting, cachexia from cancer or sepsis.

## RATIONALE FOR NUTRITIONAL SUPPLEMENTATION: THE INTERACTING PATHWAYS RESPONSIBLE FOR MITOCHONDRIAL FUNCTION.

We have found that the critical path in these interactions is the flow of energy substrates into the mitochondria through carnitine, the transfer of electrons through the complexes via CoQ10, and the modulation of the calcium pump by taurine. We have determined that the constituents of this path, namely Carnitine, CoQ10 and Taurine, to be the core constituents required to promote mitochondrial function, and act together on this critical path to provide a synergistic effect.

Other constituents may be added to these three constituents to modulate oxidative stress, which results from external factors and mitochondrial dysfunction, and which in turn promotes further dysfunction.

The added action of creatine, which is known to be deficient in cardiac failure, and of antioxidants to reduce oxidative stress, which is known to be elevated in cardiac failure, will enhance the action of the three core constituents

The addition of protein to any supplement is extremely important for obtaining the optimum benefits of these constituents in remodelling the heart.

These nutritional supplement of the present invention can be administered as oral replacements, in the form of a liquid dietary supplement, in the form of a nutritional bar, or in any other convenient form, to benefit both myocyte function and long-term survival.

## DISORDERS

Adequate energy production is essential for long term cell survival. Cellular energy production from nutrients, especially fatty acids, needs the coordinated action of a number of co-factors. Although many factors have been identified as important to proper nutrition, most patient therapy has emphasized the administration of some form of overall diet, with in some instances a vitamin or protein supplement. In heart failure the focus is usually on salt and cholesterol content.

The unique combination of the three nutrients of the present invention provide the coordinated action required for proper cellular energy production. Carnitine is critical for the transport of long chain fatty acid substrate and glucose oxidation. Coenzyme Q10 is a key. transducer for mitochondrial oxidative phosphorylation. Taurine is a key modulator of intracellular calcium. These are all important in promoting normal cell energetics.

## DETAILS OF ALTERED MITOCHONDRIAL ENERGETICS IN HEART FAILURE.

The data for mitochondrial energetic dysfunction has been clearly documented in heart failure. Consequently, the explanation of the present invention, as we believe it to operate at the date of this application, will focus on heart failure as a paradigm.

In heart failure, deficiency of carnitine is believed to promote the accumulation of toxic longchain fatty acids and imbalance between glycolysis and glucose oxidation. Deficiency of CoQ10 appears to alter electron transport, and to lead to accumulation of mitochondrial calcium, which can be corrected by the action of taurine. However, normalization of any one of the above three constituents alone will not be sufficient to provide a significant benefit to myocardial energy production in the presence of abnormalities in the other factors in the myocardial bioenergetic pathway.

## REGULATING INTRACELLULAR CALCIUM

### Taurine

The failing myocardium exhibits an increase in calcium content, and impaired movement of intracellular calcium. Intracellular calcium levels rise and fall through release and re-uptake of calcium on each contraction and relaxation of cardiac muscle cells. Chronic intracellular calcium overload leads to cell dysfunction and ultimately to the death of the patient.

Taurine (2-aminoethanesulfonic acid) is a unique amino acid, with anti-oxidant properties that has been recognized as important in the modulation of cellular calcium levels, increasing or decreasing calcium levels appropriate to the maintenance of cellular calcium homeostasis. In the heart, taurine appears to do this by affecting several myocardial membrane systems.

Taurine is found in particularly high concentrations in the heart. While Taurine is not an essential amino acid in humans, as it can be synthesized from cysteine or methionine; most taurine in humans is obtained directly through dietary sources, particularly from fish and milk.

Since cysteine will replenish not only taurine but also glutathione (an important endogenous antioxidant, as noted below), cysteine may be an important supplement for replenishing both. Cysteine, however, acts only indirectly, and administration of cysteine does not guarantee replenished taurine or glutathione levels.

Cardiac taurine concentrations have been found to be altered in heart disease, and depleted following acute ischemic injury and cardiovascular bypass surgery. Increased cytokine activity such as seen in heart failure also leads to an increased need for cysteine and taurine. Prolonged taurine depletion of the myocardium has been shown to decrease contractile force through reduction of myofibrils. Increased calcium levels in the myocyte can lead to the breakdown and loss of myofibils. Taurine depletion also renders the heart more susceptible to ischemic injury.

Taurine administration has been shown significantly reduce calcium overload and myocardial damage in a variety of heart failure models. Taurine may have a beneficial effect on cardiac arrhythmias including those associated with digitalis or catecholamine excess.

Studies of taurine administration in humans have been limited. Oral dose of I gram three times per day, given to patients suffering from congestive heart failure taurine, has been reported to be extremely well tolerated and to improve both hemodynamic state and functional capacity.

Taurine has been shown to benefit other organs including the brain, the kidney, and the liver.

## MYOCARDIAL ENERGETICS

### Carnitine

L-carnitine is an amino acid derivative (3-hydroxy-4-N-trimethylaminobutyric acid), that is essential for fatty acid oxidation and efficient glucose operation. It is integral to myocardial energy production. Carnitine deficiency has been recognized in several genetically determined metabolic abnormalities, where it is associated with the development of cardiomyopathy and skeletal muscle dysfunction. L-carnitine administration to these patients was shown to restore to a great extent cardiac and skeletal muscle function.

Carnitine deficiency may also be acquired and organ selective. The impaired heart exhibits a marked depletion (up to 50%) of myocardial carnitine levels (particularly free carnitine), with evolution of the heart disease. Plasma carnitine levels are not representative of the levels in the heart.

Carnitine also binds acyl groups and releases free CoA, removing toxic short chain acyl groups from the myocyte, and maintaining sufficient amounts of free CoA for mitochondrial function.

Carnitine has a detoxifying action. For example, in ischemic myocardium or skeletal muscle there is an accumulation of long chain acyl-CoA; these compounds are potentially toxic as they exhibit detergent-like properties and can impair mitochondrial energy production through the inhibition of a mitochondrial membrane enzyme, adenine nucleotide translocase, which transfers newly synthesized ATP from the inner mitochondria space into the cytoplasm. Carnitine protects the heart (or skeletal muscle) from the accumulation of these metabolic poisons by forming acylcarnitines, which can freely diffuse out of the cell and be eliminated through the urine.

Carnitine deficiency has also been observed in patients with chronic renal failure; and carnitine therapy has been reported to improve cardiac function for those on hemodialysis.

Body stores of 1-carnitine are supplied by both diet and via endogenous biosynthesis from trimethyllysine. L-carnitine is well tolerated and no adverse effects have been described in the literature.

Acetyl-1-carnitine and propionyl-1-carnitine are naturally occurring derivatives of 1-carnitine. The administration of those compounds has been shown to be of benefit in aging and neurodegenerative patients. Studies with L-carnitine have been limited.

L-carnitine (3 - 5 grams) or propionyl-1-carnitine (1.5 - 3.0 grams) administration has been shown to result in significant hemodynamic improvement, and an overall benefit in the functional capacity of animals and patients with heart failure or myocardial ischemia. Clinical studies have reported a reduction in cardiac damage, when 1-carnitine is taken from 4 - 12 weeks following myocardial infarction. It also appears to benefit patients who suffer from skeletal muscle ischemia manifested as intermittent claudication.

### Ubiquinone or Coenzyme Q10:

Coenzyme Q10 or ubiquinone (2,3 dimethoxy-5 methyl-6-decaprenyl benzoquinone) is believed to be very important for oxidative energy production and proper cardiac function. It plays a vital role as a rate-limiting carrier for the flow of electrons through complexes I, II and III of the mitochondrial respiratory chain. It is also a major endogenous lipophilic antioxidant and, like vitamin C, can regenerate a-tocopherol, the active form of vitamin E, by reducing the a-tocopherol radical. A deficiency of ubiquinone caused by actual loss, or through oxidation of the molecule, can result in an impairment of energy production. It is also an important component of circulating LDL particles, protecting LDL from oxidation.

Ubiquinone is actively biosynthesized with the cells, from the amino acid tyrosine and the mevalonate pathway.

Ubiquinone is widespread throughout all food groups; body stores may be partially supplied by diet. Oral absorption of ubiquinone is slow, by markedly enhanced in the presence of lipid. Only about 2 - 5 % of an oral dose is taken up by the myocardium. Side effects are virtually absent; however, asymptomatic elevations in liver enzymes (LDH, SGOT) have been described with doses of 300 mg/day.

Significantly reduced levels of myocardial ubiquinone are found in heart failure in both animal models and man.

Oral ubiquinone therapy has been shown to benefit cardiac dysfunction in a variety of animal paradigms. Oral ubiquinone also has been reported to reduce the age-associated decline in mitochondrial respiratory function in rat skeletal muscle. Trials in patients have demonstrated promising results.

**Creatine:**

Creatine phosphate (PCr) is the primary high-energy phosphate reservoir of the heart and skeletal muscle. High energy phosphate is transferred from PCr to ADP to form ATP through catalysis by creatine kinase:

$$PCr + ADP + H^+ <> ATP + Cr$$

Muscle creatine stores are maintained through biosynthesis from the endogenous precursors arginine, glycine, and methionine in the liver, pancreas and kidneys, and through the ingestion of meat and fish. Creatine is accumulated by muscle against a large concentration gradient from the blood; the transporter is understood to be driven by the extracellular/intracellular $Na^+$ electrochemical potential. There is evidence that increased adrenergic drive (a characteristic of heart failure) can decrease myocardial creatine and creatine kinase stores.

Experimental creatine depletion in animals has been shown to result in structural, metabolic and functional abnormalities in muscle. Myocardial creatine content and myocardial energetics is reduced in a wide variety of animal paradigms of heart failure. Creatine has been shown to attenuate myocardial metabolic stress in rats caused by inhibition of nitric oxide synthesis.

Hearts from patients with coronary artery disease, aortic stenosis or heart failure all show a marked reduction in total creatine (up to 50%) with an expected concomitant reduction in creatine phosphate and reduce creatine kinase. These reductions interact synergistically, decreasing myocardial capacity for ATP synthesis by up to 80%. Such an energy deficit has a significantly adverse impact on myocardial function and survival. Similar abnormalities of energy stores and production are seen in the skeletal muscles of patients of heart failure; these play an important role in compromising the functional capacity of these patients. Recently it has been reported that the myocardial PCr/ATP ratio may be a better predictor of patient mortality in dilated cardiomyopathy than left ventricular ejection fraction or the patient's functional class.

**Creatine Supplementation and Treatment**

Creatine supplementation has been shown to increase performance in situations where the availability of creatine phosphate is important. Ingestion of 3 g of creatine per day for one month (or 20 g per day for one week) increases muscle creatine content and can improve performance. Creatine supplements will increase skeletal muscle creatine and increase muscle resistance to fatigue during short-term intense exercise, where it reduces lactate accumulation. Adequate doses of creatine benefit patients with heart failure where myocardial and perhaps skeletal muscle creatine and creatine phosphate levels are depressed. The administration of a creatine supplement to patients with heart failure did not increase cardiac ejection fraction, but significantly increased not only skeletal muscle creatine phosphate but also muscle strength and endurance. Creatine supplementation would benefit patient symptom-limited performance.

**Thiamine:**

Thiamine or vitamin $B_1$ is a water-soluble vitamin which functions as a coenzyme in a variety of enzyme systems, especially those related to energy metabolism. Since Thiamine is stored only in the body in very small quantities, thiamine requirements must be met daily. Thiamine requirements are related to daily energy expenditure and metabolizable energy intake, especially carbohydrate intake. Patients with increased metabolic rates or poor intakes, such as those with heart failure, may be at increased risk for thiamine deficiency during acute illness. Thiamine deficiency results in beri beri, which can have neurological or cardiac effects. The most common symptoms are mental confusion, anorexia, muscle weakness ataxia, edema, muscle wasting, tachycardia and an enlarged heart. Thiamine deficiency leads to several major derangements of the cardiovascular system. Since thiamine deficiency will exacerbate co-existing heart failure, correction of this deficiency through supplementation has the potential to improve cardiac status in patients with congestive heart failure.

In addition to poor dietary intake and increased metabolic utilization, referred to above, patients with heart failure also may be at risk for thiamine deficiency because of their need for diuretics. Diuretics can cause increased urinary losses of thiamine even in the presence of thiamine deficiency. Patients with heart failure may be at increased risk of thiamine deficiency due to a combination of 1) poor intake resulting from anorexia and unpalatable diets, 2) hypermetabolism, and 3) enhanced excretion caused by the concurrent use of diuretics.

Thiamine deficiency is commonly present in patients on hemodialysis, in patients in intensive care units and perhaps cognitive impairment in the aged.

## REDUCING OXIDATIVE STRESS

Cells are constantly subjected to interplay between free radical injury and protective mechanisms which prevent or minimize free radical injury. Oxidative stress has been defined as a disturbance in the equilibrium between pro- and anti-oxidative systems. A number of different challenges increase oxidative stress, resulting in damage to lipids, proteins, DNA and carbohydrates.

Dietary antioxidants such as Vitamin E, Vitamin C, Cysteine and Selenium can counteract the effect of free radicals generated by external factors and by mitochondrial dysfunction. Cysteine is the precursor of glutathione, one of the most potent antioxidants present in the cell.

Until recently, it has not been widely accepted that oxidative stress can be important in the pathogenesis of cardiac disease. Recent investigation suggests that oxidative stress may be a very important contributor to the deterioration of the hypertrophied or failing myocardium.

For example, reactive oxygen species have been shown to be critical components of the apoptosis pathway; myocyte loss by apoptosis is now thought to be a significant contributor to the inexorable deterioration of the failing myocardium. The importance of oxidative stress in heart failure is not surprising because a number of factors associated with heart failure, such as increased plasma catecholamines, and cardiac sympathetic tone, microvascular reperfusion injury' cytokine stimulation and mitochondrial DNA mutations (particularly complex I) are known stimuli for free radical production and oxidative stress"'. Coenzyme Q10 and taurine (and its precursor cysteine), discussed above are important endogenous antioxidants or antioxidant precursors.

Peroxidative damage has been demonstrated in the hearts of dogs, guinea pigs and rats with heart failure due to pressure or volume overload". Vitamin E administration benefited both myocardial structure and function. We have observed decreases in the levels of glutathione peroxidase and a-tocopherol and a concomitant increase in protein oxidation in the myocardium of cardiomyopathic hamsters during the late stages of hamster cardiomyopathy ; an elevation of myocardial free radicals and lipid peroxides have also been demonstrated in this model. The administration of vitamin E appears to completely normalize these findings.

Recently, we have also demonstrated a significant increase in the plasma level of lipid peroxides and malonyldialdehyde, markers of oxidative stress, in patients suffering from congestive heart failure. The increase in oxidative stress was related to the clinical severity of heart failure with the highest levels of lipid peroxidation and malonyldialdehyde being observed in class 3 and class 4 patients. Increased free radical activity is also seen in patients on life support or in intensive care unit settings. These observations suggest that antioxidant supplements should be important additions to the therapy of heart failure and severely ill patients.

The ability to withstand peroxidative injury is partially dependent on diet. A good dietary intake of the antioxidant vitamins C and E, trace nutrient minerals such as selenium, together with adequate cysteine as a precursor for glutathione synthesis, are important for protection against free radical injury. The need for vitamin E may be increased by an increased intake of polyunsaturated fatty acids. Dietary antioxidants may reduce the risk of ischemic heart disease and the extent of myocardial infarction. Recent reports suggest an increased need for vitamin C in patients with diabetes mellitus. Oxidative stress is felt to be a major contributor to chronic neurodegenerative disease and the tissue deterioration and immune dysfunction associated with aging.

## Example 1

A nutritional supplement was prepared in the form of a protein enriched beverage having the following composition:

| Protein (casein) | 3.75 gm |
|---|---|
| Protein (whey) | 3.75 gm |

(continued)

| Carnitine | 1.5 gm |
|-----------|--------|
| Taurine | 1.5 gm |
| Creatine | 1.5 gm |
| Thiamine | 12.5 mg |
| Selenium | 25 mcg. |
| Co-Q10 | 75 mg |
| Vitamin E | 400 IU |
| Vitamin C | 250 mg |
| Cysteine | 250 mg |
| Liquid to | 125 ml |

The nutritional beverage of this example 1 is suitable for twice-daily administration, for a total of 250 ml per day.

**Example 2 - Effectiveness of Nutrient Cocktail In Vivo:**

A study was performed on cardiomyopathic hamsters to determine the effectiveness of the nutritional supplement similar to that of Example 1, and to demonstrate the feasibility of providing such a nutritional supplement. Because the metabolic rate of the hamsters was substantially different from that of human patients for whom the supplement of Example 1 was prepared, the amount of nutrients in the "cocktail" of nutrients administered to the cardiomyopathic hamsters was different from the dosage intended for humans. and that the mixture positively affected cardiac structure, function and markers of oxidative stress, deterioration of mitochondrial and myofibrillar structures in non-treated animals with improved preservation in treated animals. The results show that there are increased areas of necrosis in non-treated hearts in comparison with treated hearts. These results show that this cocktail of nutrients is effective in preserving myocyte function and structure.

We performed a pilot study in order to determine the feasibility of providing a "cocktail" of nutrients as well as their effect on indices of oxidative stress as well as on myocardial structure and function.

| Composition of Cardiac Cocktail | |
|---|---|
| L-carnitine | 300 mg/kg/day* |
| Vitamin E | 147 mg/kg/day |
| Vitamin C | 100 mg/kg/day |
| Coenzyme $Q_{10}$ | 15 mg/kg/day |
| Thiamine ($B_1$) | 100 mg/kg/day |
| Cysteine | 12 mg/kg/day |
| Selenium | 0.05 mg/day (5 mg/kg diet) |
| Taurine | 188 mg/day (18.8 g/L) |
| Creatine | 100 mg/day (1% diet) |

The nutrients were delivered in 10ml of raspberry-flavoured gelatin, commonly sold under the trade mark Jell-O. Water soluble nutrients were added directly to the cooled Jell-O while lipid soluble components were mixed with 15 ml of 20% intralipid prior to their addition to the cocktail mixture.

180-day old cardiomyopathic hamsters were started on supplementation with the formula of this example after a two-week acclimation period. 18 animals received gelatin supplemented with nutrients while a control group of 18 received the identical gelatin but without nutrients. The animals received full supplementation for eight weeks A second control group of non-diseased hamsters was maintained during this period.

At the end of this eight week period all animals were 278 days of age. At this time, 6 treated, 6 untreated and 6 non-diseased hamsters underwent the Langendorff procedure modified for hamsters, following which the hearts were preserved for electron microscopy. The remaining animals were sacrificed and blood, hearts, livers and muscle were collected for biochemistry.

Mortality - In the treated group, 2 hamsters died. Of the non-treated, diseased group, 4 hamsters died; an additional hamster was moribund at the time of study.

Appearance - The treated hamsters remained active and alert with no visible edema. In contrast, two of the non-treated animals became grossly edematous with exceptionally large black livers. The control non-treated animals were less active and appeared less bright.

Biochemistry - The heart and plasma were analysed for indices of oxidative stress.

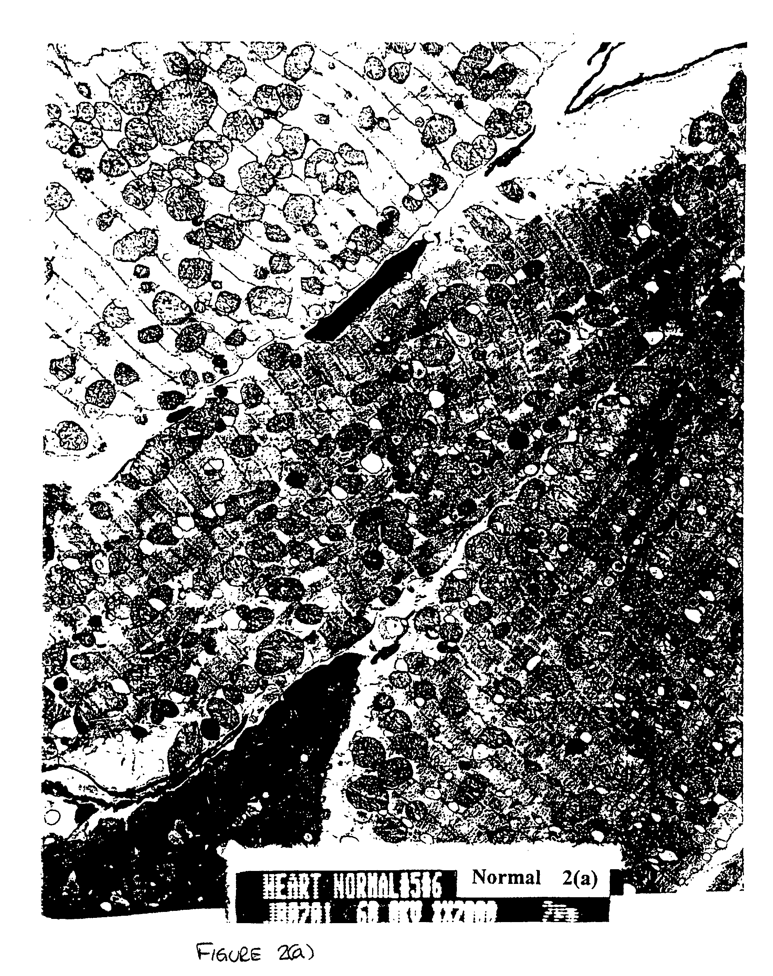

Microscopy - Electron micrographs (Figures 2 (a)-(c)) show deterioration of mitochondrial and myofibrillar structures in non-treated animals with markedly improved preservation in treated animals. In addition, there are increased areas of necrosis in non-treated hearts in comparison with treated hearts. The results achieved were superior to those projected from individual studies of the ingredients.

|  | Control Non-Treated | Treated with Supplement |
|---|---|---|
| Heart Glutathione Peroxidase Activity Units/min/mg protein | $113.05 \pm 8.20$ | $134.34 \pm 16.7$ |
| Heart Malondialydehyde µg/g wet weight | $2.00 \pm 0.25$ | $1.67 \pm 0.22$ |
| Plasma Malondialydehyde nmol/ml plasma | $0.17 \pm 0.009$ | $0.14 \pm 0.04$ |
| Plasma Glutathione Peroxidase Activity Units/min/mg protein | $5.24 \pm 0.82$ | $6.03 \pm 0.51$ |
| Ratio Heart Weight: / Body Weight | $0.008 \pm 0.0007$ | $0.007 \pm 0.0006$ |

| D. Function | Non-Treated | Treated | Normal Hamster |
|---|---|---|---|
| * Mean Pressure - Langendorff mmHg | $20.84 \pm 5.97$ | $44.17 \pm 4.55$ | $92.50 \pm 11.99$ |

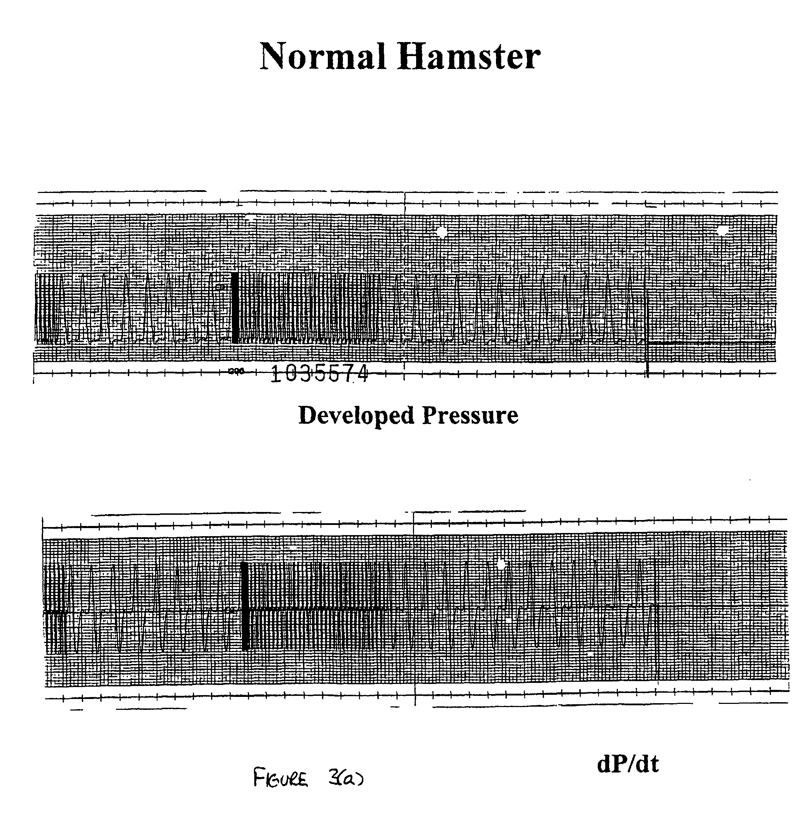

* See attached tracings (Figures 3(a)-(c))

Recently we have completed another study with the supplement and confirmed all the above results. Treatment of hamsters with a similar nutritional supplement containing only the essential three nutrients Carnitine, Co-Q10, and Taurine gives results that are similar to, but not as good as, treatment with the nutritional supplement of example 2.

## Claims

1. A nutritional supplement in a form suitable for at least once daily administration, comprising at least 0.6 g of L-Carnitine or its functional analogues selected from the group consisting of Acetyl-1-carnitine, Propionyl-1-carnitine, at least 30mg of Coenzyme Q10 (Ubiquinone) and at least 0.5 g of Taurine or a precursor of Taurine.

2. The nutritional supplement of claim 1, comprising from about 0.6 to about 6.0 g of L-Carnitine or its functional analogues, from about 30 to about 300 mg of Coenzyme Q10 (Ubiquinone) and from about 0.5 to about 5g of Taurine or its precursors.

3. The nutritional supplement of claim 1 or 2, in a protein enriched formulation.

4. The nutritional supplement of claims 1 to 3 in a formulation selected from the group consisting of a liquid dairy based product, a dehydrated dairy based product, a soy based product, and a bar.

5. The nutritional supplement of claims 1 to 4 in the form of a nutritional protein and calorie enriched bar.

6. The nutritional supplement of any of claims 1 to 5, further comprising one or more nutrients selected from the group consisting of Cysteine, Creatine, Vitamin E, Vitamin C, Selenium, and Thiamine.

7. A nutritional supplement in a form suitable for at least once daily administration comprising at least about 0.75 g of L-Carnitine or its functional analogues selected from the group consisting of Acetyl-1-carnitine, Propionyl-1-carnitine, at least 25 mg of Coenzyme Q10 (Ubiquinone) and at least about 0.75 g of Taurine or its precursors.

8. The nutritional supplement of claim 7, comprising, when present in the formulation, at least about 0.1 g of Cysteine, at least about 1 g of Creatine, at least about 100 IU of Vitamin E, at least about 100 mg Vitamin C, at least about 8 mcg of Selenium and at least about 10 mg of Thiamine.

**9.** The nutritional supplement of claim 2, comprising, when present in the formulation, from about 0.1g to about 1.0g of Cysteine, from about 0.5g to about 5g of Creatine, from about 100 to about 1200 IU of Vitamin E, from about 100mg to about 1000mg of Vitamin C, from about 8 mcg, to about 50 mcg. of Selenium, and from about 10mg to about 100 mg Thiamine.

**10.** A nutritional supplement for use in a method of treating the cardiovascular system, the central nervous system, the musculoskeletal and/or the immune system in mammals said method comprising the administration of an effective amount of the supplement according to claim 1.

**11.** The nutritional supplement of claim 10, for use in a method of treating neurodegenerative disease, immune diseases, stroke, AIDS, chronic multisystem disease, respiratory muscle fatigue such as chronic obstructive lung disease, lung or renal disease, chronic fatigue syndrome, patients on immunosuppressive drugs, cancer patients treated with drugs such as doxorubicin, wasting, cachexia from cancer or sepsis.

**12.** The nutritional supplement of claim 10, for use in a method of treating congestive heart failure.

**13.** The nutritional supplement of claims 10-12, wherein the method of treatment comprises at least once daily administration of the supplement, said daily supplement comprising at least 0.6 g of L-Carnitine or its functional analogues selected from the group consisting of Acetyl-1-carnitine, Propionyl-1-carnitine, at least 30mg of Coenzyme Q10 (Ubiquinone) and at least 0.5 g of Taurine or a precursor of Taurine.

**14.** The nutritional supplement of claim 13, comprising from about 0.6 to about 6.0 g of L-Carnitine or its functional analogues, from about 30 to about 300 mg of Coenzyme Q10 (Ubiquinone) and from about 0.5 to about 5g of Taurine or its precursors.

**15.** Use of L-Carnitine or its functional analogues selected from the group consisting of Acetyl-1-carnitine, Propionyl-1-carnitine, Coenzyme Q10 (Ubiquinone) and Taurine or a precursor of Taurine in the manufacture of a nutritional supplement for use in a method of enhancing neuromuscular or athletic performance, said method comprising administration of an effective amount of the supplement to mammals.

**16.** Use of L-Carnitine or its functional analogues selected from the group consisting of Acetyl-1-carnitine, Propionyl-1-carnitine, Coenzyme Q10 (Ubiquinone) and Taurine or a precursor of Taurine in the manufacture of a nutritional supplement for use in a method of correcting diseases, conditions and infirmities due to ageing in mammals, including humans, said method comprising the administration of an effective amount of the supplement.

**17.** Use according to claim 15 or 16, wherein the method of treatment comprises at least once daily administration of the supplement, said daily supplement comprising at least 0.6 g of L-Carnitine or its functional analogues selected from the group consisting of Acetyl-1-carnitine, Propionyl-1-carnitine, at least 30mg of Coenzyme Q10 (Ubiquinone) and at least 0.5 g of Taurine or a precursor of Taurine.

**18.** Use according to claim 17, wherein the daily supplement comprises from about 0.6 to about 6.0 g of L-Carnitine or its functional analogues, from about 30 to about 300 mg of Coenzyme Q10 (Ubiquinone) and from about 0.5 to about 5g of Taurine or its precursors.

**Patentansprüche**

**1.** Ernährungsergänzungszusammensetzung in einer für wenigstens einmal tägliche Verabreichung geeigneten Form, die wenigstens 0,6 g L-Carnitin oder seiner funktionellen Analogen, die ausgewählt sind aus der Gruppe, die aus Acetyl-L-carnitin, Propionyl-L-carnitin besteht, wenigstens 30 mg Coenzym Q10 (Ubichinon) und wenigstens 0,5 g Taurin oder eines Vorläufers von Taurin umfaßt.

**2.** Ernährungsergänzungszusammensetzung nach Anspruch 1, **dadurch gekennzeichnet, daß** sie von etwa 0,6 bis etwa 6,0 g L-Carnitin oder seiner funktionellen Analogen, von etwa 30 bis etwa 300 mg Coenzym Q10 (Ubichinon) und von etwa 0,5 bis etwa 5 g Taurin oder seiner Vorläufer umfaßt.

**3.** Ernährungsergänzungszusammensetzung nach Anspruch 1 oder 2, in einer mit Protein angereicherten Formulierung.

**4.** Ernährungsergänzungszusammensetzung nach einem der Ansprüche 1 bis 3, in einer Formulierung, die ausgewählt ist aus der Gruppe, die aus einem flüssigen Molkereiprodukt, einem dehydratisierten Molkereiprodukt, einem Sojaprodukt und einem Riegel besteht.

**5.** Ernährungsergänzungszusammensetzung nach einem der Ansprüche 1 bis 4, in der Form eines mit Ernährungsprotein und Kalorien angereicherten Riegels.

**6.** Ernährungsergänzungszusammensetzung nach einem der Ansprüche 1 bis 5, **dadurch gekennzeichnet, daß** sie weiter einen oder mehrere Nährstoffe umfaßt, die ausgewählt sind aus der Gruppe, die aus Cystein, Creatin, Vitamin E, Vitamin C, Selen und Thiamin besteht.

**7.** Ernährungsergänzungszusammensetzung in einer für wenigstens einmal tägliche Verabreichung geeigneten Form, die wenigstens etwa 0,75 g L-Carnitin oder seiner funktionellen Analogen, die ausgewählt sind aus der Gruppe, die aus Acetyl-L-carnitin, Propionyl-L-Carnitin besteht, wenigstens 25 mg Coenzym Q10 (Ubichinon) und wenigstens etwa 0,75 g Taurin oder seiner Vorläufer umfaßt.

**8.** Ernährungsergänzungszusammensetzung nach Anspruch 7, die, wenn in der Formulierung vorhanden, wenigstens etwa 0,1 g Cystein, wenigstens etwa 1 g Creatin, wenigstens etwa 100 IU Vitamin E, wenigstens etwa 100 mg Vitamin C, wenigstens etwa 8 mcg Selen und wenigstens etwa 10 mg Thiamin umfaßt.

**9.** Ernährungsergänzungszusammensetzung nach Anspruch 2, die, wenn in der Formulierung vorhanden, von etwa 0,1 g bis etwa 1,0 g Cystein, von etwa 0,5 g bis etwa 5 g Creatin, von etwa 100 bis etwa 1.200 IU Vitamin E, von etwa 100 mg bis etwa 1.000 mg Vitamin C, von etwa 8 mcg bis etwa 50 mcg Selen und von etwa 10 mg bis etwa 100 mg Thiamin umfaßt.

**10.** Ernährungsergänzungszusammensetzung zur Verwendung in einem Verfahren zur Behandlung des Herz-Kreislauf-Systems, des zentralen Nervensystems, des Muskel-Skelett- und/oder des Immun-Systems bei Säugern, wobei das Verfahren die Verabreichung einer wirksamen Menge der Ergänzungszusammensetzung nach Anspruch 1 umfaßt.

**11.** Ernährungsergänzungszusammensetzung nach Anspruch 10 zur Verwendung in einem Verfahren zur Behandlung von neurodegenerativer Erkrankung, Immunerkrankungen, Schlaganfall, AIDS, chronischer Multisystemerkrankung, Atemmuskelermüdung wie etwa chronischer obstruktiver Lungenerkrankung, Lungen- oder Nierenerkrankung, chronischem Müdigkeitssyndrom, Patienten auf Immundepressiva, Krebspatienten, die mit Medikamenten wie Doxorubicin behandelt werden, Auszehrung, Kachexie aufgrund von Krebs oder Sepsis.

**12.** Ernährungsergänzungszusammensetzung nach Anspruch 10 zur Verwendung in einem Verfahren zur Behandlung kongestiver Herzinsuffizienz.

**13.** Ernährungsergänzungszusammensetzung nach einem der Ansprüche 10 bis 12, **dadurch gekennzeichnet, daß** das Behandlungsverfahren die wenigstens einmal tägliche Verabreichung der Ergänzungszusammensetzung umfaßt, wobei besagte tägliche Ergänzungszusammensetzung wenigstens 0,6 g L-Carnitin oder seiner funktionellen Analogen, die ausgewählt sind aus der Gruppe, die aus Acetyl-L-carnitin, Propionyl-L-carnitin besteht, wenigstens 30 mg Coenzym Q10 (Ubichinon) und wenigstens 0,5 Taurin oder eines Vorläufers von Taurin umfaßt.

**14.** Ernährungsergänzungszusammensetzung nach Anspruch 13, die von etwa 0,6 bis etwa 6,0 g L-Carnitin oder seiner funktionellen Analogen, von etwa 30 bis etwa 300 mg Coenzym Q10 (Ubichinon) und von etwa 0,5 bis etwa 5 g Taurin oder seiner Vorläufer umfaßt.

**15.** Verwendung von L-Carnitin oder seinen funktionellen Analogen, die ausgewählt sind aus der Gruppe, die aus Acetyl-L-carnitin, Propionyl-L-carnitin besteht, Coenzym Q10 (Ubichinon) und Taurin oder einem Vorläufer von Taurin bei der Herstellung einer Emährungsergänzungszusammensetzung zur Verwendung in einem Verfahren zur Erhöhung der neuromuskulären oder athletischen Leistung, wobei besagtes Verfahren die Verabreichung einer wirksamen Menge der Ergänzungszusammensetzung an Säuger umfaßt.

**16.** Verwendung von L-Carnitin oder seinen funktionellen Analogen, die ausgewählt sind aus der Gruppe, die aus Acetyl-L-carnitin, Propionyl-L-carnitin besteht, Coenzym Q10 (Ubichinon) und Taurin oder einem Vorläufer von Taurin bei der Herstellung einer Ernährungsergänzungszusammensetzung zur Verwendung in einem Verfahren zum Korrigieren von Erkrankungen, Zuständen und Schwächen aufgrund von Alterung bei Säugern, einschließlich Menschen, wobei besagtes Verfahren die Verabreichung einer wirksamen Menge der Ergänzungszusammensetzung umfaßt.

17. Verwendung nach Anspruch 15 oder 16, **dadurch gekennzeichnet, daß** das Behandlungsverfahren die wenigstens einmal tägliche Verabreichung der Ergänzungszusammensetzung umfaßt, wobei besagte tägliche Ergänzungszusammensetzung wenigstens 0,6 g L-Carnitin oder seiner funktionellen Analogen, die ausgewählt sind aus der Gruppe, die aus Acetyl-L-carnitin, Propionyl-L-carnitin besteht, wenigstens 30 mg Coenzym Q10 (Ubichinon) und wenigstens 0,5 g Taurin oder eines Vorläufers von Taurin umfaßt.

18. Verwendung nach Anspruch 17, **dadurch gekennzeichnet, daß** die tägliche Ergänzungszusammensetzung von etwa 0,6 bis etwa 6,0 g L-Carnitin oder seiner funktionellen Analogen, von etwa 30 bis etwa 300 mg Coenzym Q10 (Ubichinon) und von etwa 0,5 bis etwa 5 g Taurin oder seiner Vorläufer umfaßt.

## Revendications

1. Supplément nutritionnel sous une forme convenant à une administration d'au moins une fois par jour, comprenant au moins 0,6 g de L-carnitine ou de ses analogues fonctionnels choisis dans le groupe consistant en acétyl-1-carnitine, propionyl-1-carnitine. au moins 30 mg de coenzyme Q10 (ubiquinone) et au moins 0,5 g de taurine ou d'un précurseur de taurine.

2. Supplément nutritionnel de la revendication 1, comprenant d'environ 0,6 à environ 6,0 g de L-carnitine ou de ses analogues fonctionnels, d'environ 30 à environ 300 mg de coenzyme Q10 (ubiquinone) et d'environ 0,5 à environ 5 g de taurine ou de ses précurseurs.

3. Supplément nutritionnel de la revendication 1 ou 2, dans une formulation enrichie en protéines.

4. Supplément nutritionnel des revendications 1 à 3 dans une formulation choisie dans le groupe consistant en un produit à base de laitage liquide, un produit à base de laitage déshydraté, un produit à base de soja et une barre.

5. Supplément nutritionnel des revendications 1 à 4 sous la forme d'une barre nutritionnelle enrichie en protéines et en calories.

6. Supplément nutritionnel de l'une quelconque des revendications 1 à 5, comprenant en outre un ou plusieurs nutriments choisis dans le groupe consistant en cystéine, créatine, vitamine E, vitamine C, sélénium et thiamine.

7. Supplément nutritionnel sous une forme convenant à une administration d'au moins une fois par jour, comprenant au moins environ 0,75 g de L-carnitine ou de ses analogues fonctionnels choisi dans le groupe consistant en acétyl-1-carnitine, propionyl-1-carnitine, au moins 25 mg de coenzyme Q10 (ubiquinone) et au moins environ 0,75 g de taurine ou de ses précurseurs.

8. Supplément nutritionnel de la revendication 7, comprenant, lorsqu'ils sont présents dans la formulation, au moins environ 0,1 g de cystéine, au moins environ 1 g de créatine, au moins environ 100 UI de vitamine E, au moins environ 100 mg de vitamine C, au moins environ 8 mcg de sélénium et au moins environ 10 mg de thiamine.

9. Supplément nutritionnel de la revendication 2, comprenant, lorsqu'ils sont présents dans la formulation, d'environ 0,1 g à environ 1.0 g de cystéine, d'environ 0.5 g à environ 5 g de créatine, d'environ 100 à environ 1200 UI de vitamine E. d'environ 100 mg à environ 1000 mg de vitamine C, d'environ 8 mcg à environ 50 mcg de sélénium et d'environ 10 mg à environ 100 mg de thiamine.

10. Supplément nutritionnel destiné à être utilisé dans une méthode de traitement du système cardiovasculaire, du système nerveux central, du système musculo-squelettique et/ou du système immunitaire chez des mammifères, ladite méthode comprenant l'administration d'une quantité efficace du supplément selon la revendication 1.

11. Supplément nutritionnel de la revendication 10, destiné à être utilisé dans une méthode de traitement d'une maladie neurodégénérative, de maladies immunitaires, d'une attaque, du SIDA, d'une maladie multisystémique chronique, d'une fatigue musculaire respiratoire telle qu'une bronchopneumopathie chronique obstructive, d'une maladie pulmonaire ou rénale, d'un syndrome de fatigue chronique, de patients sous médicaments immunosuppresseurs, de patients cancéreux traités par des médicaments tels que la doxorubicine, d'un amaigrissement, d'une cachexie provenant d'un cancer ou d'une septicémie.

12. Supplément nutritionnel de la revendication 10, destiné à être utilisé dans une méthode pour le traitement d'une insuffisance cardiaque congestive.

13. Supplément nutritionnel des revendications 10-12, pour lequel la méthode de traitement comprend l'administration au moins une fois par jour du supplément, ledit supplément quotidien comprenant au moins 0,6 g de L-carnitine ou de ses analogues fonctionnels choisis dans le groupe consistant en acétyl-1-carnitine, propionyl-1-carnitine, au moins 30 mg de coenzyme Q10 (ubiquinone) et au moins 0,5 g de taurine ou d'un précurseur de taurine.

14. Supplément nutritionnel de la revendication 13, comprenant d'environ 0,6 à environ 6,0 g de L-carnitine ou de ses analogues fonctionnels, d'environ 30 à environ 300 mg de coenzyme Q10 (ubiquinone) et d'environ 0,5 à environ 5 g de taurine ou de ses précurseurs.

15. Utilisation de L-carnitine ou de ses analogues fonctionnels choisis dans le groupe consistant en acétyl-1-carnitine, propionyl-1-carnitine, de coenzyme Q10 (ubiquinone) et de taurine ou d'un précurseur de taurine dans la fabrication d'un supplément nutritionnel destiné à être utilisé dans une méthode pour augmenter une performance neuromusculaire ou athlétique, ladite méthode comprenant l'administration d'une quantité efficace du supplément à des mammifères.

16. Utilisation de L-carnitine ou de ses analogues fonctionnels choisis dans le groupe consistant en acétyl-1-carnitine, propionyl-1-carnitine, de coenzyme Q10 (ubiquinone) et de taurine ou d'un précurseur de taurine dans la fabrication d'un supplément nutritionnel destiné à être utilisé dans une méthode pour corriger des maladies, des états et des infirmités dus au vieillissement chez des mammifères, incluant des humains, ladite méthode comprenant l'administration d'une quantité efficace du supplément.

17. Utilisation selon la revendication 15 ou 16, dans laquelle la méthode de traitement comprend l'administration au moins une fois par jour du supplément, ledit supplément quotidien comprenant au moins 0,6 g de L-carnitine ou de ses analogues fonctionnels choisis dans le groupe consistant en acétyl-1-carnitine, propionyl-1-carnitine, au moins 30 mg de coenzyme Q10 (ubiquinone) et au moins 0,5 g de taurine ou d'un précurseur de taurine.

18. Utilisation selon la revendication 17, dans laquelle le supplément quotidien comprend d'environ 0,6 à environ 6,0 g de L-carnitine ou de ses analogues fonctionnels, d'environ 30 à environ 300 mg de coenzyme Q10 (ubiquinone) et d'environ 0,5 à environ 5 g de taurine ou de ses précurseurs.

Figure 1.

Interaction of Nutrients in Cell Metabolism

©MJSKI

HEART NORMAL#586    Normal    2(a)

FIGURE 2a)

Cardiomyopathic Untreated 2(b)

Figure 2(b)

HEART T2‖1   Cardiomyopathic Treated   2(c)

Figure 2(c)

# Normal Hamster

**Developed Pressure**

FIGURE 3(a)                                    **dP/dt**

# Cardiomyopathic Hamster

# Untreated

**Developed Pressure**

**dP/dt**

Figure 3 (b)

# Cardiomyopathic Hamster

# Cocktail

**Developed Pressure**

**dP/dt**

Figure 3(c)